# EUROPÄISCHE PATENTSCHRIFT

(11) **EP 2 135 512 B1**
(45) Veröffentlichungstag und Bekanntmachung des Hinweises auf die Patenterteilung: **16.06.2010**
(21) Anmeldenummer: 08158574.7
(22) Anmeldetag: 19.06.2008
(51) Int. Cl.: A23L 1/00, A23L 2/00, A61K 8/365, A61K 9/08, A61K 31/19, A61K 31/216, A61K 31/353, A61K 47/12

(54) **Zusammensetzung auf der Basis von Äpfelsäure, Kaffeesäure, einem Flavan-3-ol und einem Anthocyan und ihre Verwendung in Lebensmitteln und Medizin**
Compound based on malic acid, caffeic acid, a flavan-3-ol and an anthocyane and its use in food and medicine
Composition à base d'acide malique, d'acide de café, de flavane-3-ol et d'un anthocyane et leur utilisation dans des produits alimentaires et en médecine

(43) Veröffentlichungstag der Anmeldung: 23.12.2009
(73) Patentinhaber: Muin, Ali, Dr., 1120 Wien (AT)
(72) Erfinder: Muin, Ali, Dr., 1120 Wien (AT)
(74) Vertreter: HOFFMANN EITLE

(56) Entgegenhaltungen:
- WO-A-02/38181
- WO-A-98/58540
- WO-A-2006/128032
- CH-A- 308 692
- DE-A1- 19 831 306
- DE-A1-102007 028 508
- US-A- 4 927 651
- US-A1- 2004 063 648
- US-A1- 2008 069 779

## Beschreibung

Die vorliegende Erfindung betrifft eine (pharmazeutische) Zusammensetzung, die Äpfelsäure (A), Kaffeesäure (B), mindestens eine Verbindung aus der Gruppe der Flavan-3-ole (C) und mindestens eine Verbindung aus der Gruppe der Anthocyane (D) umfasst. Insbesondere betrifft die vorliegende Erfindung die Verwendung der (pharmazeutischen) Zusammensetzung zur Stabilisierung von Lösungen von Akalisalzen des Penicillin G, die Verwendung der (pharmazeutischen) Zusammensetzung als Nahrungsergänzungsmittel und die Verwendung der (pharmazeutischen) Zusammensetzung in einem Verfahren zur prophylaktischen und therapeutischen Behandlung von Krebs. Darüber hinaus betrifft die vorliegende Erfindung ein Nahrungsmittelprodukt, Erfrischungsgetränke, eine Wund- und Heilsalbe, eine Haar-Lösung/Haarspray und eine Anti-Aging Creme, die die (pharmazeutische) Zusammensetzung enthalten. Ein weiterer Gegenstand der Erfindung ist eine Zusammensetzung, die die obige (pharmazeutische) Zusammensetzung und zusätzlich Penicillin G Natrium enthält.

### TECHNISCHER HINTERGRUND

Penicillin G ist ein bekanntes Antibiotikum der folgenden Struktur:

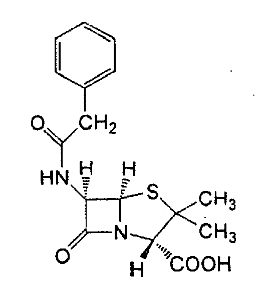

Da Penicillin G in trockener, kristalliner Form schlecht wasserlöslich und zudem nicht Magensäure-resistent ist, werden vorwiegend die in Wasser und Alkohol gut löslichen Alkalisalze als Arzneistoffe eingesetzt. Dies ist auch eine wichtige Voraussetzung, um Penicillin G intravenös verabreichen zu können. Die Stabilität der Alkalisalze von Penicillin G in Wasser und Alkohol ist jedoch stark von pH und der Temperatur abhängig und bei Raumtemperatur wird Penicillin G bei einem pH unter 4 oder über 9 rasch inaktiviert. So wird z.B. in einem Artikel von Neftel et al. (British Journal of Hematology 54(2); 255-260, 1983) beschrieben, dass der Penicillinverlust in einer Penicillinlösung bei 20 °C in 24 Stunden 38 % und bei 37 °C bereits 50 % beträgt. Es wird in diesem Artikel auch beschrieben, dass bei Temperaturen von 56 °C, wie sie im Sommer in heißen Gegenden in Autos vorkommen können, der Verlust sogar auf 66 % innerhalb von drei Stunden ansteigt. Deshalb sollte eine Lösung von Penicillin G bei Raumtemperatur maximal 24 Stunden gelagert werden, im Kühlschrank maximal eine Woche und im Tiefkühlschrank maximal 12 Wochen wie von Papich in Kirk's Current Veterinary Therapy XII - Small Animal Practice, WB Sounders Company, Philadelphia (USA), 7th Edition; S.194-199, 1995 beschrieben wird. Wie in einem weiteren Artikel von Green und Watson (Infectious Diseases in the Dog and Cat, WB Sounders Company, Philadelphia (USA), 2nd Edition; S.185-204, 1999) beschrieben, sollten Lösungen für den intravenösen Gebrauch innerhalb von 24 Stunden verbraucht werden, da die Abbauprodukte zu einem großen Teil für Nebenwirkungen verantwortlich sind und Allergien auslösen können.

Es wäre demnach wünschenswert, eine Penicillinlösung bereitzustellen, die bei Temperaturen oberhalb der Raumtemperatur über mehrere Wochen haltbar ist, d.h. ihre antibakterielle Wirkung nicht einbüßt und keine Abbauprodukte hervorbringt, die ungewünschte Nebenwirkungen hervorbringen und Allergien auslösen können.

Darüber hinaus wäre es wünschenswert, dass Mittel zur Stabilisierung von Alkalisalzen von Penicillin G in Lösung weitere physiologisch vorteilhafte Wirkungen zeigen, die in der Medizin und als Nahrungsmittel genutzt werden können.

### AUFGABEN DER VORLIEGENDEN ERFINDUNG

Es ist eine technische Aufgabe der Erfindung, eine Zusammensetzung bereitzustellen, die in der Lage ist, Penicillin G in wässriger Lösung zu stabilisieren, sodass es über einen längeren Zeitraum bei Temperaturen über Raumtemperatur haltbar ist, seine Wirkung aufgrund von Zersetzung des Penicillins nicht verliert und keine Abbauprodukte entstehen, die unerwünschte Nebenwirkungen, wie Allergieausbildung, auf den Körper haben.

Es ist zudem eine weitere Aufgabe der Erfindung, eine Zusammensetzung bereitzustellen, die darüber hinaus eine konstitutionsfördernde Wirkung beim Menschen zeigt und auch zur prophylaktischen und therapeutischen Behandlung von Krebs verwendet werden kann.

US 2004/0063648 A1 betrifft ein Verfahren zur Behandlung, Vorbeugung oder Inhibierung von Krebs durch Verabreichen mindestens einer Polyphenolverbindung und mindestens eines Inhibitors für reaktive Sauerstoffspezies. In längeren Listen für geeignete Polyphenolverbindungen werden u. a. Anthocyane, Catechine, Epigallocatechine, Pelargonidin, Cyanidin, Delphinidin, Peonidin, Malvidin und Kaffeesäure angeführt. Eine spezielle Kombination dieser Verbindungen wird nicht gelehrt. Vorzugsweise ist die Polyphenolverbindung Quercetin, Rutin, Genistin oder Trans-Resveratrol. In einem Beispiel zur Bewertung des Einflusses dieser bevorzugten Polyphenolverbindungen auf das Wachstum von Krebszellen wird ferner die Zugabe von Penicillin-G zu Dulbecco's Modified Eagle's Medium beschrieben.

WO 02/38181 A2 betrifft Arzneimittel, mit einer mikrobiziden Zusammensetzung aus wenigstens zwei Aromastoffen und die Verwendung dieser Zusammensetzung zur Herstellung von dekontaminativen und/oder regenerativen Mitteln für die Behandlung von Mensch und Tier. Beispielhafte Zusammensetzungen dieses Arzeimittels können jeweils einzeln 10% Apfelsäure, 10% Kaffeesäure oder 30% Catechine enthalten. Penicillin G wird in diesem Dokument nicht erwähnt.

DE 198 31 306 A1 betrifft ein Verfahren und ein Additiv sowie dessen Verwendung zur Haltbarkeitsverbesserung und/oder Stabilisierung von mikrobiell verderblichen Produkten durch Zusatz von Additiven. Das Additiv ist ein Gemisch aus a) Benzylalkohol oder Polyphenol und b) wenigstens einem nicht-alkoholischen vorzugsweise hydrophilen weiteren Aromastoff, ausgenommen Polyphenol, in einem speziellen Mischungsverhältnis. In längeren Listen für einsetzbare Polyphenole finden sich Anthocyanidine, Catechine und Kaffeesäure unter zahlreichen weiteren Komponenten. Als weitere Komponente des Additivs werden auch Säuren, u. a. Apfelsäure, erwähnt. Penicillin G wird in diesem Dokument nicht erwähnt.

DE 10 2007 028 508 A1 betrifft kosmetische Deodorantien und Antitranspirantien, die zur Verbesserung der Lagerstabilität, insbesondere der Farb- und/oder Geruchsstabilität, eine Kombination aus mindestens einem ausgewählten Antioxidans und mindestens einem Komplexbildner enthalten. Unter zahlreichen Beispielen für Antioxidantien finden sich auch Flavonoide, Kaffeesäure und Epigallocatechin. Penicillin G wird in diesem Dokument nicht erwähnt.

### KURZE BESCHREIBUNG DER ERFINDUNG

Die oben erwähnten Aufgaben werden durch die folgenden erfindungsgemäßen Gegenstände gelöst:

Eine Zusammensetzung (Z), die Äpfelsäure (A), Kaffeesäure (B), mindestens eine Verbindung aus der Gruppe der Flavan-3-ole (C) und mindestens eine Verbindung aus der Gruppe der Anthocyane (D) umfasst.

Die erfindungsgemäße Zusammensetzung kann eine pharmazeutische Zusammensetzung sein.

Die erfindungsgemäße (pharmazeutischen) Zusammensetzung (Z) kann zur Herstellung eines Medikaments zur prophylaktischen und therapeutischen Behandlung von Krebs verwendet werden.

Ein weiterer Gegenstand der vorliegenden Erfindung ist die Verwendung der erfindungsgemäßen (pharmazeutischen) Zusammensetzung (Z) als Nahrungsergänzungsmittel.

Die vorliegende Erfindung betrifft auch ein Nahrungsmittelprodukt, das die oben genannte erfindungsgemäße (pharmazeutische) Zusammensetzung (Z) umfasst.

Zudem betrifft die vorliegende Erfindung die Verwendung der erfindungsgemäßen (pharmazeutischen) Zusammensetzung (Z) zur Stabilisierung von gelösten Alkalisalzen des Penicillin G, insbesondere Penicillin G Natrium.

Ein weiterer Gegenstand der vorliegenden Erfindung ist eine wässrige Lösung (W), die die erfindungsgemäße (pharmazeutische) Zusammensetzung (Z) enthält.

Die vorliegende Erfindung betrifft auch eine wässrige Lösung (Wp) und eine wässrige Infusionslösung (W_{I}), die die erfindungsgemäße (pharmazeutische) Zusammensetzung (Z) und ein Alkalisalz des Penicillin G, insbesondere Penicillin G Natrium umfassen, und ihre Verwendung in einem Verfahren zur prophylaktischen und therapeutischen Behandlung von bakteriellen Infektionen und zur prophylaktischen und therapeutischen Behandlung von Krebs.

### DETAILLIERTE BESCHREIBUNG DER ERFINDUNG

Die erfindungsgemäße Zusammensetzung betrifft in einer ersten Ausführungsform eine Zusammensetzung, die Äpfelsäure (A), Kaffeesäure (B), mindestens eine Verbindung aus der Gruppe der Flavan-3-ole (C) und mindestens eine Verbindung aus der Gruppe der Anthocyane (D) umfasst.

Zudem kann die Zusammensetzung in einer bevorzugten Ausführungsform zusätzlich mindestens eine weitere phenolische Verbindung (E) enthalten.

In einer weiteren bevorzugten Ausführungsform können die oben genannten Zusammensetzungen zusätzlich mindestens eine weitere organische Säure (F) enthalten, die keine phenolische Verbindung ist.

Eine weitere Ausführungsform der Erfindung ist eine Zusammensetzung der oben genannten Ausführungsformen, die zusätzlich mindestens eines der folgenden Metalle (G) in der Form eines physiologisch verträglichen Metallsalzes enthält: Kalium, Calcium, Kupfer, Eisen, Zink, Magnesium, Natrium und Mangan.

Des Weiteren kann die Zusammensetzung der obigen Ausführungsformen zusätzlich mindestens einen Zucker (H) enthalten. Mögliche Zucker schliessen Mono-, Di- und OligoSaccharide ein.

### DIE VIER HAUPTBESTANDTEILE (A) BIS (D) DER ERFINDUNGSGEMÄßEN (PHARMAZEUTISCHEN) ZUSAMMENSETZUNG (Z)

Die vier Hauptbestandteile der erfindungsgemäßen (pharmazeutischen) Zusammensetzung sind Äpfelsäure (A), Kaffeesäure (B), mindestens eine Verbindung aus der Gruppe der Flavan-3-ole (C) und mindestens eine Verbindung aus der Gruppe der Anthocyane (D). Die erfindungsgemäße Zusammensetzung ist nicht auf diese vier Hauptbestandteile beschränkt und kann weitere Bestandteile enthalten, die weiter unten beschrieben sind.

Äpfelsäure (A), auch 2-Hydroxybernsteinsäure genannt, besitzt die Summenformel C₄H₆O₅ und hat eine relative Molmasse von 134,09 g pro Mol. Die Äpfelsäure kann sowohl in der D- als auch in der L-Form vorliegen. Beide Formen können erfindungsgemäß verwendet werden, allerdings ist die ausschließlich in der Natur vorkommende L-Form erfindungsgemäß bevorzugt.

Kaffeesäure (B), die auch 3,4-Dihydroxyzimtsäure genannt wird, hat die Summenformel C₉H₈O₄ und besitzt eine molare Masse von 180,16 g pro Mol. Ein weiterer geläufiger Name ist Trans-3-(3,4-Dihydroxyphenyl)-Propensäure.

Die Verbindung (C) aus der Gruppe der Flavan-3-ole schließt u.a. (+)-Catechin, (-)-Catechin, (+)-Epicatechin, (-)-Epicatechin, (+)-Gallocatechin, (-)-Epigallocatechin, (-)-Epigallocatechin gallat, (-)-Epicatechin 3-gallat, Theoflavin, Theoflavin-3-gallat, Theoflavin-3'-gallat, Theoflavin-3,3'-digallat und Thearubigin ein. Bevorzugt in der vorliegenden Erfindung sind (+)-Catechin und (-)-Epicatechin. Beide können in Kombination oder einzeln eingesetzt werden. (+)-Catechin ist besonders bevorzugt.

Die Gruppe der Anthocyane unterteilt sich in die Anthocyanidine und Anthocyanine. Die Anthocyanidine stellen Aglycone dar und die Anthocyanine Glycoside. In der erfindungsgemäßen Zusammensetzung können eine oder mehrere Verbindungen aus der Gruppe der Anthocyanidine und/oder der Anthocyanine verwendet werden. Der Zusammenhang zwischen Anthocyanen und Anthocyanidinen besteht darin, dass die Anthocyane ein in 3- oder 5-Stellung glycosidiertes Anthocyanidin darstellen. Deshalb werden die Anthocyanidine auch als Aglycone bezeichnet. Die vorliegende Erfindung schließt die Verwendung von Anthocyanidinen mit der folgenden Grundstruktur ein:

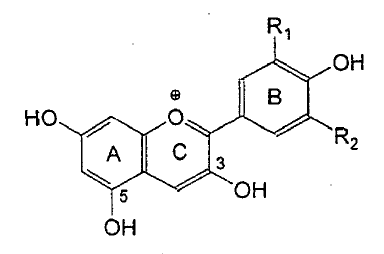

**Tab. 1 zeigt erfindungsgemäß bevorzugte Anthocyandidine und deren Substitutionsmuster:**

| **Anthocyanidin** | **R₁** | **R₂** |
|---|---|---|
| Cyanidin | OH | H |
| Delphinidin | OH | OH |
| Malvidin | OCH₃ | OCH₃ |
| Pelargonidin | H | H |
| Peonidin | OCH₃ | H |
| Petunidin | OH | OCH₃ |

Ist die obige Formel der Anthocyanidine in 3- oder 5-Stellung glycosidiert, spricht man von der Gruppe der Anthocyane. In der vorliegenden Erfindung können vorzugsweise jegliche in 3- und 5-Stellung substituierte Anthocyanidine eingesetzt werden. Die Anthocyane der in Tab. 1 aufgeführten Anthocyanidine werden erfindungsgemäß bervorzugt. Besonders bevorzugt als Anthocyan (D) ist in der vorliegenden Erfindung das Malvidin-3-glucosid.

### DIE OPTIONALEN BESTANDTEILE (E) BIS (H) DER ERFINDUNGSGEMÄßEN (PHARMAZEUTISCHEN) ZUSAMMENSETZUNG (Z)

Die erfindungsgemäße (pharmazeutische) Zusammensetzung kann vorzugsweise zusätzlich zu den oben genannten vier Hauptbestandteilen (A) bis (D) noch mindestens eine weitere phenolische Verbindung (E) enthalten. In der vorliegenden Erfindung versteht man unter dem Begriff "phenolische Verbindung" jegliche Verbindung, die eine oder mehrere Phenolgruppen enthält. Phenolische Verbindungen schließen Monophenole, Polyphenole, Phenolsäuren, Hydroxyzimtsäuren und Tyrosole, Derivate der Tyrosole und Ester der Tyrosole ein. Monophenole schließen sämtliche Verbindungen ein, die eine Phenolgruppe im Molekül enthalten, wie z.B. Apiol, Carnosol, Carvacrol und Dillapiol ein. Die Gruppe der Polyphenole, die auch als Flavonoide bezeichnet wird, schließt z.B. die Flavonole, Flavonone und Flavone ein. Die Gruppe der Phenolsäuren schließt vorzugsweise Ellagsäure, Gallussäure, Salicylsäure, Gerbsäure, Vanillin, Kapsaicin und Kurcumin ein. Besonders bevorzugt unter den Phenolsäuren ist die Gallussäure. Die Hydroxyzimtsäuren schließen vorzugsweise die Chlorogensäure, auch 3,4-Dihydroxyzinnamoylchinasäure genannt, die Zimtsäure, die Ferulasäure und Cumarin ein.

Die Tyrosole, Tyrosolderivate und deren Ester schließen z.B. Tyrosol, Hydroxytyrosol, Olecanthal und Oleuropein und Derivate davon und deren Ester ein. Besonders bevorzugt in der vorliegenden Erfindung ist Tyrosol.

Weitere Phenolsäuren, die einzeln oder in Kombination mit den oben genannten Phenolsäuren in der vorliegenden Erfindung verwendet werden können, sind Caftarsäure, Cis-Cutarsäure, Trans-Cutarsäure und Fertarsäure.

Für die phenolische Verbindung (E), die zusätzlich zu den Hauptbestandteilen (A) bis (D) in der erfindungsgemäßen Zusammensetzung enthalten sein kann, gilt die Maßgabe, dass die Kaffeesäure, Verbindungen aus der Gruppe der Flavan-3-ole und die Anthocyane ausgenommen sind.

Die erfindungsgemäße (pharmazeutische) Zusammensetzung (Z) kann außerdem vorzugsweise zusätzlich mindestens eine weitere organische Säure (F) enthalten, die keine phenolische Verbindung ist. Für diese weitere organische Säure (F), die keine phenolische Verbindung ist, gilt die Maßgabe, dass sie nicht Äpfelsäure ist. Des Weiteren kann jegliche organische Säure, die keine Phenolgruppe enthält, erfindungsgemäß verwendet werden. Unter diesen organischen Säuren ist die Ascorbinsäure bevorzugt. Die organische Säure ist vorzugsweise nicht Weinsäure, d.h. Weinsäure ist in der erfindungsgemäßen (pharmazeutischen) Zusammensetzung vorzugsweise nicht vorhanden.

In einer weiteren erfindungsgemäßen Ausführungsform kann jegliche der oben genannten Zusammensetzungen mindestens eines der folgenden Metalle (G) in der Form eines Metallsalzes enthalten: Kalium, Calcium, Kupfer, Eisen, Zink, Magnesium, Natrium und Mangan.

Alle denkbaren physiologisch verträglichen Metallsalze der oben genannten Metalle können verwendet werden. Bevorzugte Salze dieser Metalle sind organische Salze, wie Acetate, Propionate, Tartrate, Oxalate, und anorganische Salze wie Sulfate, Chloride und Phosphate. Die Auswahl geeigneter Salze erfolgt auch im Hinblick auf eine ausreichende Wasserlöslichkeit der Zusammensetzung aller enthaltenen Metalle.

Ein weiterer Bestandteil der oben genannten erfindungsgemäßen (pharmazeutischen) Zusammensetzung kann auch ein Zucker sein. Der Zucker kann ein Mono-, Di- oder Oligosaccharide sein. Di- bzw. Oligosaccharide (gr.: oligos - wenig), sind Kohlenhydrate (Zucker), die aus zwei bzw. mehreren gleichen oder verschiedenen Monosacchariden aufgebaut sind, und durch glykosidische Bindungen miteinander verbunden sind. Entsprechend der Anzahl der vorhandenen Monosaccharideinheiten umfassen Oligosaccharide Tri-, Tetra-, Pentasacchariden usw., die sowohl linear als auch verzweigt sein können. Jeder dieser Zucker kann einzeln, aber auch in Kombination von zwei oder mehreren eingesetzt werden. Unter Oligosacchariden in der vorliegenden Erfindung versteht man Verbindungen mit bis zu 20 Monomereinheiten.

Erfindungsgemäß bevorzugte Zucker sind D-Glucose und D-Fructose. In einer besonders bevorzugten Ausführungsform werden D-Glucose und D-Fructose in Kombination verwendet.

### ANTEILE DER KOMPONENTEN IN DER ERFINDUNGSGEMÄßEN (PHARMAZEUTISCHEN) ZUSAMMENSETZUNG (Z)

Die erfindungsgemäße Zusammensetzung einer der oben genannten Ausführungsformen enthält die Verbindungen (A) bis (D) in den folgenden Mengen:
Äpfelsäure(A): vorzugsweise mindestens 30, besonders bevorzugt 43 bis 73 Gew.-Teile,
Kaffeesäure(B): vorzugsweise mindestens 0.001, besonders bevorzugt 0.005 bis 0.1 Gew.-Teile,
Flavan-3-ole(C): vorzugsweise mindestens 0.005, besonders bevorzugt 0.015 bis 0.3 Gew.-Teile und
Anthocyane(D): vorzugsweise mindestens 0.05, besonders bevorzugt 0.1 bis 2 Gew.-Teile,
bezogen auf 100 Gew.-Teile aller Bestandteile mit Ausnahme von Zucker und ggf. vorhandenem Lösungsmittel.

In einer bevorzugten Ausführungsform enthält die erfindungsgemäße Zusammensetzung die folgenden optionalen Bestandteile: mindestens eine weitere phenolische Verbindung (E), mindestens eine weitere organische Säure (F), die keine phenolische Säure ist, und mindestens ein Metall (G) in der Form eines Metallsalz der oben erwähnten Metalle.

Die optionalen Bestandteile (E) bis (G) liegen vorzugsweise in folgenden Mengen vor:
Phenolische Verbindung(E): vorzugsweise bis zu 1,5,
besonders bevorzugt 0,3 bis 0,9 Gew.-Teile,
Organische Säure(F): vorzugsweise bis zu 15,
besonders bevorzugt 3 bis 9 Gew.-Teile,
Metall(G): vorzugsweise bis zu 30,
besonders bevorzugt 10 bis 20 Gew.-Teile,
bezogen auf 100 Gew.-Teile aller Bestandteile mit Ausnahme von Zucker und ggf. vorhandenem Lösungsmittel.

Bevorzugte Ausführungsformen der wesentlichen und optionalen Bestandteile werden im Folgenden erläutert:
Äpfelsäure (A) liegt in der erfindungsgemäßen (pharmazeutischen) Zusammensetzung in einer Menge von mindestens 30 Gew.-Teilen, vorzugsweise 43 bis 73 Gew.-Teilen, noch stärker bevorzugt 50 bis 60 Gew.-Teilen und besonders bevorzugt 53 bis 57 Gew.-Teilen vor, bezogen auf 100 Gew.-Teile aller Bestandteile mit Ausnahme von Zucker und ggf. vorhandenem Lösungsmittel.

Kaffeesäure (B) liegt in der erfindungsgemäßen Zusammensetzung in einem Anteil von mindestens 0.001, vorzugsweise 0,005 bis 0,1 Gew.-Teilen, besonders bevorzugt 0,01 bis 0,08 Gew.-Teilen und noch stärker bevorzugt 0,03 bis 0,05 Gew.-Teilen vor, bezogen auf 100 Gew.-Teilen aller Bestandteile mit Ausnahme von Zucker und ggf. vorhandenem Lösungsmittel.

Die Verbindung aus der Gruppe der Flavan-3-ole (C) liegt in der erfindungsgemäßen (pharmazeutischen) Zusammensetzung in einem Anteil von mindestens 0.005 Gew.-Teilen, vorzugsweise 0,015 bis 0,3 Gew.-Teilen, besonders bevorzugt 0,05 bis 0,2 Gew.-Teilen und noch stärker bevorzugt 0,1 bis 0,18 Gew.-Teilen vor, bezogen auf 100 Gew.-Teile aller Bestandteile mit Ausnahme von Zucker und ggf. vorhandenem Lösungsmittel.

Die Verbindung aus der Gruppe der Anthocyane (D) liegt in der erfindungsgemäßen (pharmazeutischen) Zusammensetzung in einer Menge von mindestens 0,05 Gew.-Teilen, vorzugsweise 0,1 bis 2 Gew.-Teilen, besonders bevorzugt 0,5 bis 1,5 Gew.-Teilen und noch stärker bevorzugt 0,8 bis 1,3 Gew.-Teilen vor, bezogen auf 100 Gew.-Teile aller Bestandteile mit Ausnahme von Zucker und ggf. vorhandenem Lösungsmittel.

Sofern die erfindungsgemäße (pharmazeutische) Zusammensetzung eine weitere phenolische Verbindung (E) enthält, liegt diese z.B. in einer Menge von bis zu 1,5 Gew.-Teilen, vorzugsweise im Bereich von 0,3 bis 0,9 Gew.-Teilen, besonders bevorzugt 0,4 bis 0,8 Gew.-Teilen und noch stärker bevorzugt 0,5 bis 0,6 Gew.-Teilen vor, bezogen auf 100 Gew.-Teile aller Bestandteile mit Ausnahme von Zucker und ggf. vorhandenem Lösungsmittel.

In der erfindungsgemäßen Zusammensetzung kann der Anteil an Caftarsäure 0,03 bis 0,09 Gew.-Teile, der Anteil an Cis-Cutarsäure zwischen 0 und 0,01 Gew.-Teile, der Anteil an Trans-Cutarsäure zwischen 0,001 und 0,01 Gew.-Teile, der Anteil an Fertarsäure 0,001 bis 0,02 Gew.-Teile, der Anteil an Gallussäure 0,3 bis 0,6 Gew.-Teile und der Anteil an Tyrosol 0,03 bis 0,07 Gew.-Teile sein, bezogen auf 100 Gew.-Teile aller Bestandteile mit Ausnahme von Zucker und ggf. vorhandenem Lösungsmittel. Besonders bevorzugt wird Gallussäure alleine als phenolische Verbindung (E) oder in Kombination mit Tyrosol und/oder Caftarsäure verwendet. Noch stärker bevorzugt wird als phenolische Verbindung (E) eine Mischung aus Caftarsäure, Cis-Cutarsäure, Trans-Cutarsäure, Fertarsäure, Gallussäure und Tyrosol eingesetzt.

Enthält die erfindungsgemäße (pharmazeutische) Zusammensetzung eine weitere organische Säure (F), die keine phenolische Verbindung ist, so liegt diese z.B. in einem Anteil von bis zu 15 Gew.-Teilen, vorzugsweise 3 bis 9 Gew.-Teilen, besonders bevorzugt 4 bis 8 Gew.-Teilen und noch stärker bevorzugt 5 bis 7 Gew.-Teilen vor, bezogen auf 100 Gew.-Teile aller Bestandteile mit Ausnahme von Zucker und ggf. vorhandenem Lösungsmittel.

Wenn die erfindungsgemäße (pharmazeutische) Zusammensetzung mindestens eines der obengenannten Metalle (G) in der Form eines Metallsalzes enthält, so ist der Anteil des Metalls (G) z.B. bis zu 30 Gew.-Teile, vorzugsweise 10 bis 20 Gew.-Teile, besonders bevorzugt 12 bis 18 Gew.-Teile und noch stärker bevorzugt 14 bis 16 Gew.-Teile, bezogen auf 100 Gew.-Teile aller Bestandteile mit Ausnahme von Zucker und ggf. vorhandenem Lösungsmittel. Kalium kann in einer Menge von 10 bis 15 Gew.-Teilen, Calcium in einer Menge von 0,3 bis 1,6 Gew.-Teilen, Kupfer in einer Menge von 0,0005 bis 0,004 Gew.-Teilen, Eisen in einer Menge von 0,01 bis 0,05 Gew.-Teilen, Zink in einer Menge von 0,001 bis 0,01 Gew.-Teilen, Magnesium in einer Menge von 0,5 bis 1,5 Gew.-Teilen, Natrium in einer Menge von 0,05 bis 0,2 Gew.-Teilen und Mangan in einer Menge von 0,001 bis 0,005 Gew.-Teilen in der erfindungsgemäßen (pharmazeutischen) Zusammensetzung vorliegen. Liegt ein Metallsalz (G) dieser Metalle vor, so ist ein Kaliumsalz entweder einzeln oder in Kombination mit einem oder mehreren der anderen Metallsalze bevorzugt. Besonders bevorzugt wird das Kaliumsalz in Kombination mit einem Eisensalz, einem Zinksalz und/oder einem Mangansalz eingesetzt. Noch stärker bevorzugt werden alle oben genannten Metalle in der Form von Metallsalzen gleichzeitig im Bereich der bevorzugten Mengen eingesetzt.

Enthält die Zusammensetzung Zucker (H), so liegt dieser z.B. in einer Menge von 60 bis 95 Gew.-%, bevorzugt 75 bis 92 Gew.-%, besonders bevorzugt 88 bis 90 Gew.-% vor, bezogen auf alle Bestandteile der Zusammensetzung. D.h., der Anteil der übrigen Bestandteile ist z.B. 5 bis 40 Gew.-%, bevorzugt 8 bis 25 Gew.-% und besonders bevorzugt 10 bis 12 Gew.-% bezogen auf alle Bestandteile der Zusammensetzung. Der Anteil der übrigen Bestandteile kann sich aus den Hauptbestandteilen (A) bis (D) alleine zusammensetzen oder in einer bevorzugten Ausführungsform aus den vier Hauptbestandteilen (A) bis (D) und einer oder mehrerer der optionalen Komponenten (E) bis (G) oder ggf. weiteren Bestandteilen zusammen. In einer bevorzugten Ausführungsform besteht der Zuckeranteil aus einer Mischung aus D-Glucose und D-Fructose. Das Verhältnis von Glucose und Fructose liegt im Bereich von 1/0,5 bis 1/2, bevorzugt 1/0,8 bis 1/1,5 und besonders bevorzugt 1/1,01.

In einer stark bevorzugten Ausführungsform der vorliegenden Erfindung enthält die erfindungsgemäße (pharmazeutische) Zusammensetzung 43 bis 46 Gewichtsteile Glucose, 43 bis 46 Gewichtsteile Fructose, 5 bis 7 Gewichtsteile Äpfelsäure, 2 x 10⁻³ bis 4 x 10⁻³ Gewichtsteile Kaffeesäure, 0,5 bis 0,7 Gewichtsteile Ascorbinsäure, 5,0 bis 7,0 x 10⁻³ Caftarsäure, 0 bis 1,2 x 10⁻³ Gewichtsteile Cis-Cutarsäure, 0 bis 1,4 x 10⁻³ Gewichtsteile Trans-Cutarsäure, 0 bis 1,4 x 10⁻³ Gewichtsteile Fertarsäure, 40 x 10⁻³ bis 55 x 10⁻³ Gewichtsteile Gallussäure, 4 x 10⁻³ bis 7 x 10⁻³ Gewichtsteile Tyrosol, 1 x 10⁻³ bis 3 x 10⁻³ Gewichtsteile Catechin, 6 x 10⁻³ bis 10 x 10⁻³ Gewichtsteile Epicatechin, 30 x 10⁻³ bis 60 x 10⁻³ Malvidin-3-Glucosid, 1 bis 2 Gewichtsteile Kalium, 88 x 10⁻³ bis 100 x 10⁻³ Gewichtsteile Calcium, 0,1 x 10⁻³ bis 0,5 x 10⁻³ Gewichtsteile Kupfer, 2 x 10⁻³ bis 5 x 10⁻³ Gewichtsteile Eisen, 0,30 x 10⁻³ bis 0,60 x 10⁻³ Gewichtsteile Zink, 70 x 10⁻³ bis 90 x 10⁻³ Gewichtsteile Magnesium, 10 x 10⁻³ bis 20 x 10⁻³ Gewichtsteile Natrium und 0,2 x 10⁻³ bis 0,4 x 10⁻³ Gewichtsteile Mangan, bezogen auf 100 Gew.-Teile der gesamten Zusammensetzung inklusive Zucker.

### DIE VERWENDUNG DER ERFINDUNGSGEMÄßEN (PHARMAZEUTISCHEN) ZUSAMMENSETZUNG (Z)

Eine weitere Ausführungsform betrifft die erfindungsgemäße pharmazeutische Zusammensetzung zur Verwendung zur prophylaktischen und therapeutischen Behandlung von Krebs. Unter prophylaktischer Behandlung versteht man in der vorliegenden Erfindung die vorbeugende Behandlung vor der Entstehung von Krebs. Dies schließt auch eine gesundheitsfördernde Wirkung und die damit verbundene Krebsprävention ein. Unter dem Begriff therapeutische Behandlung von Krebs versteht man die Behandlung zur vollständigen oder teilweisen Zerstörung von Krebszellen im Blut oder Tumorzellen durch Zellauflösung.

### DIE ERFINDUNGSGEMÄßE WÄSSRIGE LÖSUNG (W)

Eine weitere erfindungsgemäße Ausführungsform ist eine wässrige Lösung (W), die die erfindungsgemäße (pharmazeutische) Zusammensetzung gelöst in destilliertem Wasser enthält. Somit kann die wässrige Lösung (W) eine pharmazeutische Lösung sein.

### ANTEIL DER KOMPONENTEN DER ERFINDUNGSGEMÄßEN (PHARMAZEUTISCHEN) ZUSAMMENSETZUNG IN DER ERFINDUNGSGEMÄßEN WÄSSRIGEN LÖSUNG (W)

Die Summe der Bestandteile Äpfelsäure (A), Kaffesäure (B), Flavan-3-ol (C), Anthocyan (D) und der optionalen Bestandteile phenolische Verbindung (E), organische Säure (F), Metall (G) und Zucker (H) in der erfindungsgemäßen wässrigen Lösung beträgt vorzugsweise mindestens 0,03 Gew.-%, stärker bevorzugt 0,03 bis 30 Gew.-%, noch stärker bevorzugt 0,4 bis 16 Gew.-%, noch stärker bevorzugt 1 bis 14 Gew.-%, noch stärker bevorzugt 6 bis 12 und am stärksten bevorzugt 8 bis 10 Gew.-% bezogen auf das Gewicht des Wassers.

Die erfindungsgemäße wässrige Lösung enthält mindestens 3 g/l Äpfelsäure (A), bevorzugt 4,3 bis 7,3 g/l, besonders bevorzugt 5,0 bis 6,6 g/l. Die Lösung enthält mindestens 0,5 mg/l Kaffeesäure (B), bevorzugt 0,5 bis 10 mg/l, besonders bevorzugt 2,0 bis 4 mg/l. Die Verbindung aus der Gruppe Flavan-3-ole (C) liegt in der wässrigen Lösung in einer Menge von mindestens 1,5 mg/l, bevorzugt 1,5 bis 30 mg/l, besonders bevorzugt 6,0 bis 15,0 mg/l und außerordentlich bevorzugt 8 bis 10 mg/l vor. Die Lösung enthält zudem die Verbindung aus der Gruppe der Anthocyane (D) in einer Mindestmenge von 10 mg/l, bevorzugt 10 bis 200 mg/l, besonders bevorzugt 15 bis 100 mg/l und außerordentlich bevorzugt 30 bis 55 mg/l.

Enthält die wässrige Zusammensetzung die folgenden optionalen Bestandteile(E), (F), (G) und (H), so liegen sie in den folgenden Mengen vor:
Phenolische Verbindung (E): vorzugsweise bis zu 90 mg/l, besonders bevorzugt im Bereich von 30 bis 90 mg/l, noch stärker bevorzugt 55 bis 67 mg/l;
Organische Säure(F): vorzugsweise bis zu 900 mg/l, besonders bevorzugt im Bereich von 300 bis 900 mg/l, noch stärker bevorzugt 5 bis 7 mg/l;
Metall(G): vorzugsweise bis zu 1.6 g/l, besonders bevorzugt 1.0 bis 2.0 g/l, noch stärker bevorzugt 1.4 bis 1.6 mg/l;
Zucker(H): vorzugsweise bis zu 95 g/l, besonders bevorzugt im Bereich von 60 bis 95 g/l, noch stärker bevorzugt 75 bis 92 g/l, außerordentlich bevorzugt 88 bis 90 g/l.

Die erfindungsgemäße wässrige Lösung (W) enthält in einer stark bevorzugten Ausführungsform 40,0 bis 43,0 g/l D-Glucose, 40 bis 43,3 g/l D-Fructose, 5,0 bis 6,6 g/l L-Äpfelsäure, 500 bis 660 mg/l Ascorbinsäure, ggf. 5 bis 7 mg/l Caftarsäure, 0 oder ggf. mehr als 0 (z.B. 0,01 mg), aber maximal 1 mg/l Cis-Cutarsäure, ggf. 0,5 bis 1,5 mg/l Trans-Cutarsäure, ggf. 0,5 bis 1,5 mg/l Fertarsäure, 2,5 bis 3,5 mg/l Kaffeesäure, 43 bis 51 mg/l Gallussäure, 4 bis 6 mg/l Tyrosol, 1,5 bis 2,5 mg/l Catechin, 6 bis 8 mg/l Epicatechin, 38 bis 46 mg/l Malvidin-3-Glucosid, 1200 bis 1500 mg/l Kalium, 80 bis 100 mg/l Calcium, 0,15 bis 0,25 mg/l Kupfer, 2,5 bis 4,0 mg/l Eisen, 0,3 bis 0,5 mg/l Zink, 70 bis 80 mg/l Magnesium, 10 bis 20 mg/l Natrium und 0,2 bis 0,4 mg/l Mangan.

### VERWENDUNG DER ERFINDUNGSGEMÄßEN WÄSSRIGEN LÖSUNG (W)

Die erfindungsgemäße wässrige Lösung kann zur Herstellung einer stabilen Penicillin G-haltigen Infusionslösung verwendet werden, die in einem Verfahren zur therapeutischen und prophylaktischen Behandlung von Krebs eingesetzt werden kann.

### DIE ERFINDUNGSGEMÄßE WÄSSRIGE LÖSUNG (Wp)

Eine noch weitere Ausführungsform der vorliegenden Erfindung ist eine wässrige Lösung (Wp), die die erfindungsgemäße (pharmazeutische) Zusammensetzung (Z) und ein Alkalisalz des Penicillin G, insbesondere Penicillin G Natrium, gelöst in destilliertem Wasser, enthält. Somit kann die wässrige Lösung (Wp) eine pharmazeutische Lösung sein. Ein Penicillin G Alkalisalz kann in Form der Lösung (Wp) stabil gelagert werden.

Penicillin G wird auch Benzylpenicillin genannt und liegt als solches in Form der freien Säure vor. Unter einem Alkalisalz von Penicillin G versteht man das Alkalicarboxylat des Penicillin G. Als "Alkali" kann Natrium oder Kalium eingesetzt werden. Vorzugsweise wird das Natriumsalz eingesetzt.

Die erfindungsgemäße wässrige Lösung (Wp) enthält die erfindungsgemäße (pharmazeutische) Zusammensetzung und ein Alkalisalz des Penicillin G, insbesondere Penicillin G Natrium, vorzugsweise in den folgenden Mengen:
- die zuvor beschriebene erfindungsgemäße (pharmazeutische) Zusammensetzung ohne Zuckeranteil: vorzugsweise bis zu 8 g/l, stärker bevorzugt 2 bis 7 g/l, noch stärker bevorzugt 3 bis 6 g/l, am stärksten bevorzugt 4 bis 5 g/l;
- Zucker(H): falls vorhanden in einer Menge von vorzugsweise bis zu 47,5 g/l, besonders bevorzugt im Bereich von 30 bis 47,5 g/l, noch stärker bevorzugt 37,5 bis 46 g/l, außerordentlich bevorzugt 42 bis 45 g/l;
- Alkalisalz des Penicillin G, insbesondere Penicillin G Natrium: vorzugsweise bis zu 40 g/l, besonders bevorzugt 20 bis 35 g/l, stärker bevorzugt 25 bis 35 g/l, am stärksten bevorzugt 28 bis 32 g/l.

### DIE VERWENDUNG DER ERFINDUNGSGEMÄßEN (PHARMAZEUTISCHEN) WÄSSRIGEN LÖSUNG (Wp)

Die wässrige Lösung (Wp) kann in einem Verfahren zur prophylaktischen oder therapeutischen Behandlung von bakteriellen Infektionen und ferner zur prophylaktischen oder therapeutischen Behandlung von Krebs verwendet werden. In anderen Worten betrifft diese Ausführungsform die Verwendung der wässrigen Lösung (W_{P}), welche die erfindungsgemäße (pharmazeutische) Zusammensetzung und zusätzlich ein Alkalisalz des Penicillin G, insbesondere Penicillin G Natrium, umfasst, zur Herstellung eines Medikaments zur prophylaktischen oder therapeutischen Behandlung von bakteriellen Infektionen und ferner zur prophylaktischen oder therapeutischen Behandlung von Krebs. Unter einer bakteriellen Infektion versteht man die Erkrankung eines Menschen oder Tiers durch Bakterienbefall.

### DIE ERFINDUNGSGEMÄßE WÄSSRIGE INFUSIONSLÖSUNG (W_{I})

Eine noch weitere Ausführungsform der vorliegenden Erfindung ist eine wässrige Infusionslösung (W_{I}), welche die erfindungsgemäße (pharmazeutische) Zusammensetzung (Z), ein Alkalisalz des Penicillin G, insbesondere Penicillin G Natrium, und zusätzlich Natriumchlorid, gelöst in destilliertem Wasser, enthält. Somit kann die wässrige Lösung (W_{I}) eine pharmazeutische Lösung sein.

Die erfindungsgemäße wässrige Lösung (W_{I}) enthält die erfindungsgemäße (pharmazeutische) Zusammensetzung, ein Alkalisalz des Penicillin G, insbesondere Penicillin G Natrium, und zusätzlich Natriumchlorid, vorzugsweise in den folgenden Mengen:
- die zuvor beschriebene erfindungsgemäße (pharmazeutische) Zusammensetzung ohne Zuckeranteil: vorzugsweise bis zu 4 g/l, stärker bevorzugt 0,25 bis 4 g/l, noch stärker bevorzugt 0,5 bis 3 g/l, am stärksten bevorzugt 1 bis 2 g/l;
- Zucker(H): falls vorhanden in einer Menge von vorzugsweise bis zu 24 g/l, besonders bevorzugt im Bereich von 3,75 bis 24 g/l, noch stärker bevorzugt 10 bis 23 g/l, außerordentlich bevorzugt 18 bis 22,5 g/l;
- Alkalisalz des Penicillin G, insbesondere Penicillin G Natrium: vorzugsweise bis zu 20 g/l, besonders bevorzugt 2,5 bis 20 g/l, stärker bevorzugt 7 bis 18 g/l, am stärksten bevorzugt 14 bis 16 g/l;
- zusätzliches Natriumchlorid: vorzugsweise bis zu 9 g/l, stärker bevorzugt 4 bis 8,5 g/l, noch stärker bevorzugt 4,4 bis 8 g/l.

### DIE VERWENDUNG DER ERFINDUNGSGEMÄßEN (PHARMAZEUTISCHEN) WÄSSRIGEN INFUSIONSLÖSUNG (W_{I})

Die wässrige Lösung (W_{I}) kann in einem Verfahren zur prophylaktischen oder therapeutischen Behandlung von bakteriellen Infektionen und ferner zur prophylaktischen oder therapeutischen Behandlung von Krebs verwendet werden. In anderen Worten betrifft diese Ausführungsform die Verwendung der wässrigen Lösung (W_{I}), welche die erfindungsgemäße (pharmazeutische) Zusammensetzung, ein Alkalisalz des Penicillin G, insbesondere Penicillin G Natrium, und zusätzlich Natriumchlorid umfasst, zur Herstellung eines Medikaments zur prophylaktischen oder therapeutischen Behandlung von bakteriellen Infektionen und ferner zur prophylaktischen oder therapeutischen Behandlung von Krebs.

### WEITERE AUSFÜHRUNGSFORMEN DER VORLIEGENDEN ERFINDUNG

In einer weiteren Ausführungsform kann die erfindungsgemäße (pharmazeutische) Zusammensetzung (Z) auch als Nahrungsergänzungsmittel verwendet werden. Nahrungsergänzungsmittel sind Stoffe, die Nahrungsmitteln oder Lebensmitteln zugesetzt werden, um deren Eigenschaften den Wünschen der Konsumenten und Lebensmittelhersteller anzupassen. Sie dienen zur erhöhten Versorgung des menschlichen Stoffwechsels mit bestimmten Nähr- oder Wirkstoffen und können somit auch als auch Arzneimittel fungieren.

Ein Nahrungsmittelprodukt im Sinne der vorliegenden Erfindung, das die erfindungsgemäße (pharmazeutische) Zusammensetzung (Z) enthält, kann ein festes oder flüssiges Nahrungsmittelprodukt sein.

Feste Nahrungsmittelprodukte schließen Süßwaren-Produkte, Produkte, die verarbeitetes Getreide oder Kartoffeln enthalten oder Produkte, die aus verarbeitetem Obst, Gemüse, Fleisch und Fisch bestehen, Kekse, Kuchen, Brot, Kaugummi, Produkte, die aus verarbeiteten Nüssen oder Samen bestehen, ein. Bevorzugte Süßwaren-Produkte sind Karamell, Marmelade, Eiscreme oder Schokoladenprodukte. Das feste Nahrungsmittelprodukt enthält die erfindungsgemäße (pharmazeutische) Zusammensetzung (Z) vorzugsweise in einer Menge von bis zu 50 Gew.-%, z.B. 0.1 bis 45 Gew.%, stärker bevorzugt von 1 bis 50 Gew.-%, noch stärker bevorzugt von 5 bis 40 Gew.-%, noch stärker bevorzugt von 10 bis 35 Gew.-%, noch stärker bevorzugt von 15 bis 25 Gew.-% und am stärksten bevorzugt von 18 bis 22 Gew.-%.

Flüssige Nahrungsmittelprodukte schließen alkoholische Getränke, Erfrischungsgetränke, wie Mineralwasser, Soft-Drinks, Fruchtsäfte und Gemüsesäfte, Milch oder Milchprodukte, Tee, Kaffee, Kakao und eine Mischung aus den vorher genannten Getränken ein. Das flüssige Nahrungsmittelprodukt enthält die erfindungsgemäße (pharmazeutische) Zusammensetzung vorzugsweise in einer Menge von bis zu 30 Gew.-%, z.B. 0.1 bis 30 Gew.%, stärker bevorzugt 1 bis 28 Gew.-%, noch stärker bevorzugt 6 bis 25 Gew.-%, noch stärker bevorzugt 10 bis 20 Gew.-% und am stärksten bevorzugt 13 bis 16 Gew.-%.

Eine weitere Ausführungsform der vorliegenden Erfindung ist eine Zusammensetzung, die die erfindungsgemäße (pharmazeutische) Zusammensetzung (Z) und Gingko- oder Ginseng-Extrakt enthält. Eine Mischung aus der erfindungsgemäßen Zusammensetzung und Gingko-Extrakt wird zur Behandlung und Prophylaxe von Demenz, Arteriosklerose und cardiovaskulären Erkrankungen eingesetzt. Eine Mischung aus der erfindungsgemäßen Zusammensetzung (Z) und Ginseng-Extrakt wirkt immunstimulierend, erhöht das Leistungs- und Konzentrationsvermögen und wird bei Libidomangel, Müdigkeits- und Schwächegefühl, psychischem und physischem Stress, Rekonvaleszenz, Schlafstörungen und Tinnitus eingesetzt.

In einer weiteren Ausführungsform kann die erfindungsgemäße Zusammensetzung auch in einer Wund- und Heilsalbe eingesetzt werden. Vorzugsweise enthält eine solche Wund- und Heilsalbe die erfindungsgemäße Zusammensetzung (Z), Wasser, ein Alkalisalz des Penicillin G, insbesondere Penicillin G Natrium, und Bacitrazin Zink oder Inotyol Puder.

In einer weiteren Ausführungsform liegt die erfindungsgemäße Zusammensetzung in der Form eines Lyophilisats vor. Ein Lyophilisat ist ein in gefrorenem Zustand Gefrier- oder Sublimations-getrocknetes Produkt.

Eine weitere Ausführungsform der vorliegenden Erfindung ist die Verwendung der erfindungsgemäßen Lösung (W) zur Herstellung einer Haar-Lösung bzw. eines Haarsprays. Hierbei liegt die Lösung (W) zu einer 70%igen medizinischen Alkohollösung im Verhältnisbereich von 1:2 bis 1:10, besonders bevorzugt im Verhältnis 1:6 vor.

Zudem kann die erfindungsgemäße Lösung (W) auch zur Herstellung einer Anti-Aging Creme verwendet werden. Neben der erfindungsgemäßen Lösung (W) kann die Anti-Aging Creme vorzugsweise die Vitamine A, E und D und Hyaluronsäure enthalten.

### VERABREICHUNGSFORMEN UND DOSIERUNG

Die erfindungsgemäße (pharmazeutische) Zusammensetzung (Z) kann in der Form von Kapseln oder Tabletten für die orale Gabe bereitgestellt werden. Zudem kann die Zusammensetzung auch für die intravenöse oder intramuskuläre Verabreichung in Wasser gelöst vorliegen.

Die Kapseln oder Tabletten zur oralen Verabreichung enthalten die erfindungsgemäße (pharmazeutische) Zusammensetzung (Z) in einer der obigen Ausführungsformen und mindestens einen Füllstoff. Der in der vorliegenden Erfindung verwendete Füllstoff kann ein für Arzneistoffe gängiger Füllstoff sein. Füllstoffe sind inerte und physiologisch gut verträgliche Stoffe zur Volumenauffüllung bei niedrig dosierten Wirkstoffen oder zur besseren Dispergierung von Wirkstoffen in den Arzneiformen, z.B. zur Verhinderung eines Zusammenlagerns einzelner Wirkstoffpartikel. Erfindungsgemäß bevorzugte Füllstoffe sind Milchzucker, Cellulosepulver, Mannitol, Calciumdiphosphat und Stärke. Wenn eine Kapsel verwendet wird, wird vorzugsweise eine Gelatinekapsel herkömmlicher Art verwendet. Gelatinekapseln haben den Vorteil, dass sie im sauren Milieu des Magens nicht löslich sind, sodass eine Resorption der wirksamen Zusammensetzung erst im Darm stattfindet.

Kapseln oder Tabletten für die orale Verabreichung enthalten vorzugsweise zwischen 15 und 25 Gewichtsprozent der erfindungsgemäßen Zusammensetzung (Z) und 75 bis 85 Gewichtsprozent des Füllstoffs. In einer besonders bevorzugten Ausführungsform enthalten die Kapseln oder Tabletten 20 Gewichtsprozent erfindungsgemäße(pharmazeutische) Zusammensetzung und 80 Gewichtsprozent Füllstoff. Die Gesamtmenge einer Kapselfüllung oder einer Tablette beträgt z.B. 500 mg, worin die erfindungsgemäße (pharmazeutische) Zusammensetzung in einer Menge von 80 bis 120 mg und der Füllstoff in einer Menge von 380 bis 420 mg vorliegen. Besonders bevorzugt liegen die Zusammensetzung zu 100 mg und der Füllstoff zu 400 mg vor. In der Regel werden zwei dieser Kapseln täglich (vorzugsweise morgens und abends) verabreicht. Die Dosierung kann jedoch je nach Bedarf auch höher sein und z.B. bei Erwachsenen zwischen 150 und 600 mg pro Tag betragen.

Enthält die Kapsel zusätzlich zur erfindungsgemäßen Zusammensetzung (Z) und dem Füllstoff noch Gingko- bzw. Ginseng-Extrakt, so enthält sie die erfindungsgemäße Zusammensetzung vorzugsweise zwischen 15 und 25 Gew.-%, den Gingko- bzw. Ginseng-Extrakt zwischen 5 und 10 Gew.-% und den Füllstoff zwischen 70 und 80 Gew.-%. Die Gesamtmenge einer solchen Kapselfüllung beträgt z.B. 540 mg, worin die erfindungsgemäße Zusammensetzung in einer Menge von 80 bis 120 mg, der Gingko- bzw. Ginseng-Extrakt in einer Menge von 30 bis 50 mg und der Füllstoff in einer Menge von 380 bis 420 mg vorliegen.

Wird die erfindungsgemäße (pharmazeutische) Zusammensetzung als Nahrungsergänzungsmittel eingesetzt, so ist vorzugsweise eine um 1/3 höhere Dosis als bei den Kapseln oder Tabletten zu verabreichen, da ein Teil der Wirkstoffe durch das saure Milieu im Magen zersetzt wird, d.h. ca. 200 bis 800 mg.

Die erfindungsgemäße wässrige Lösung (Wp), die die erfindungsgemäße (pharmazeutische) Zusammensetzung und ein gelöstes Alkalisalz des Penicillin G, insbesondere Penicillin G Natrium enthält, wird vorzugsweise zur Herstellung einer Infusionslösung verwendet, die intravenös oder intramuskulär verabreicht werden kann.

Die erfindungsgemäße wässrige Infusionslösung (W_{I}), die die erfindungsgemäße (pharmazeutische) Zusammensetzung, ein gelöstes Alkalisalz des Penicillin G, insbesondere Penicillin G Natrium, und zusätzlich Natriumchlorid enthält, wird vorzugsweise intravenös oder intramuskulär verabreicht.

### WIRKUNGEN DER ERFINDUNG

Nach derzeitigem Kenntnisstand spielen die folgenden Wirkungen bei der vorliegenden Erfindung eine Rolle.

Die Wirkungsweise der erfindungsgemäßen (pharmazeutischen) Zusammensetzung zur prophylaktischen und therapeutischen Behandlung von Krebs (Tumorzellen und Leukämie) beruht vermutlich zum einen darauf, dass durch Eiweißspaltung die Membranen der Krebszellen aufgelöst werden, so dass der Tumor bis auf das Bindegewebe (Nekrose) schrumpft und anschließend leichter operativ entfernt werden kann. Zum anderen hat die erfindungsgemäße (pharmazeutische) Zusammensetzung vermutlich eine steigernde Wirkung auf Abwehrkomplexe, wie z.B. Fresszellen. Außerdem wird durch die erfindungsgemäße (pharmazeutische) Zusammensetzung vermutlich der Stoffwechsel beschleunigt, so dass die Abfallprodukte der aufgelösten Tumorzellen leichter ausgeschieden werden können. Außerdem unterstützen die in der Zusammensetzung vorhandenen Antioxidantien vermutlich den Aufbau von Körper-, Nerven-, Muskel-, Gewebs-, und Gehirnzellen, was eine leistungssteigernde Wirkung hervorruft. Die erfindungsgemäße (pharmazeutische) Zusammensetzung ist zudem nicht toxisch.

### BEISPIELE

### Beispiel 1:

Es wurde eine wässrige Lösung durch Auflösen einzelner Bestandteile der erfindungsgemäßen Zusammensetzung hergestellt, die folgende Verbindungen in den nachstehenden Mengen enthielt:

| | |
|---|---|
| D-Glucose | 42,0 g/l |
| D-Fructose | 42,3 g/l |
| L-Äpfelsäure | 5,8 g/l |
| Ascorbinsäure | 580 mg/l |
| Caftarsäure | 6,0 mg/l |
| Kaffeesäure | 3,0 mg/l |
| Gallussäure | 47,0 mg/l |
| Tyrosol | 5,0 mg/l |
| Catechin | 2,0 mg/l |
| Epi-Catechin | 7,0 mg/l |
| Malvidin-3-Glucosid | 42,0 mg/l |
| Kalium | 1.340 mg/l |
| Calcium | 91,0 mg/l |
| Kupfer | 0,20 mg/l |
| Eisen | 3,27 mg/l |
| Zink | 0,41 mg/l |
| Magnesium | 75,0 mg/l |
| Natrium | 14,0 mg/l |
| Mangan | 0,29 mg/l |

Die oben genannten Substanzen wurden käuflich erworben und die Metalle wurden in der Form käuflich erhältlicher und physiologisch verträglicher Salze eingesetzt.

### Beispiel 2:

Es wurden 200 ml einer wässrigen Lösung (Wp) durch Mischen von 100 ml der Lösung gemäß Beispiel 1 und 100 ml einer wässrigen Lösung von 6 g Penicillin G Natrium in destilliertem Wasser erhalten. Somit enthielt diese Lösung Penicillin G Natrium in einer Konzentration von 30 g/l.

### Beispiel 3: Stabilitätsuntersuchung

Jeweils eine 200 ml Probe der in Beispiel 2 hergestellten Lösung wurde bei 4 bis 8 °C im Kühlschrank aufbewahrt. Nach jeweils 7 Tagen, 14 Tagen, 28 Tagen, 8 Wochen und 12 Wochen wurde ein HPLC Chromatogramm aufgenommen und mit einem HPLC Chromatogramm der Lösung direkt nach der Herstellung verglichen. Es wurde festgestellt, dass sich die Zusammensetzung auch nach 12 Wochen nicht veränderte, d.h. das Penicillin G blieb über den gesamten Zeitraum unverändert stabil in der Lösung. Es wurde zudem während der gesamten Lagerungszeit kein Niederschlag von Feststoffen in der Lösung festgestellt, wohingegen eine Vergleichslösung von Penicillin G Natrium in destilliertem Wasser, welche die erfindungsgemäße Zusammensetzung nicht enthielt, sich trübte und eine erhebliche Menge an Niederschlag bildete.

Auf die gleiche Weise wurde die Stabilität der Lösung nach 10 Tagen bei Lagerung bei Raumtemperatur (21 °C, näherungsweise 40 % relative Luftfeuchtigkeit) durchgeführt. Die Auswertung der HPLC-DAD Chromatogramme ergab, dass Penicillin G unverändert stabil in Lösung vorlag.

### HPLC VERFAHREN:

Die Chromatogramme wurden mit einem HPLC-DAD Chromatographen aufgenommen. Sie wurden bei verschiedenen Wellenlängen aufgenommen. Die HPLC Trennung wurde durch Umkehrphasen-Chromatographie durchgeführt.

### HPLC-Parameter:

| | |
|---|---|
| Säulenmaterial: | Spezielle RP-stationäre Phase für hydrophileverbindungen 4 µm, 250 x 4,6 mm; |
| | |
| Flußgeschwindigkeit: | 1 ml/min; |
| | |
| Elutionsmittel: | Gradientenmodus |
| | Elutionsmittel A: Wasser/ACN |
| | (Acetonitril)/Phosphorsäure 1N |
| | Elutionsmittel B: |
| | Wasser/ACN/Phosphorsäure 1N; |
| | |
| Einspritzvolumen: | 2 µl; |
| | |
| Detektion: | UV bei 254 nm, 200 nm, 230 nm, 280 nm und 350 nm; |
| | |
| Stopzeit: | 25 min; |

### Beispiel 4: Herstellung einer Gelatine-Kapsel mit der erfindungsgemäßen Zusammensetzung

Eine Gelatine-Kapsel wurde hergestellt, indem 100 mg der folgenden Zusammensetzung und 400 mg Cellulosepulver in einem Mörser vermischt und in eine Gelatine-Membran eingekapselt wurden:

Die erfindungsgemäße Zusammensetzung enthielt die folgenden Bestandteile in Gew.-Teilen:

| | | |
|---|---|---|
| D-Glucose | 45,5 | Gew.-Teile |
| D-Fructose | 45,8 | Gew.-Teile |
| L-Äpfelsäure | 6,3 | Gew.-Teile |
| Ascorbinsäure | 0,638 | Gew.-Teile |
| Caftarsäure | 6,4 x 10⁻³ | Gew.-Teile |
| Kaffeesäure | 3,2 x 10⁻³ | Gew.-Teile |
| Gallussäure | 50,9 x 10⁻³ | Gew. -Teile |
| Tyrosol | 5,5 x 10⁻³ | Gew.-Teile |
| Catechin | 2,15 x 10⁻³ | Gew.-Teile |
| Epicatechin | 7,58 x 10⁻³ | Gew.-Teile |
| Malvidin-3-Glucosid | 45,89 x 10⁻³ | Gew.-Teile |
| Kalium | 1,45 | Gew.-Teile |
| Calcium | 90,08 x 10⁻³ | Gew.-Teile |
| Kupfer | 0,21 x 10⁻³ | Gew.-Teile |
| Eisen | 3,54 x 10⁻³ | Gew.-Teile |
| Zink | 0,44 x 10⁻³ | Gew. -Teile |
| Magnesium | 80,2 x 10⁻³ | Gew.-Teile |
| Natrium | 15,6 x 1.0⁻³ | Gew.-Teile |
| Mangan | 0,31 x 10⁻³ | Gew.-Teile |

### Beispiel 5: Herstellen von Infusionslösungen

Es wurden drei verschiedene Infusionslösungen (Infusionslösung 1 bis 3) hergestellt. Hierzu wurde die in Beispiel 2 hergestellte Lösung mit 0,9%iger NaCl-Lösung in den folgenden Mengen bei Raumtemperatur vermischt:
Infusionslösung 1: 100 ml der in Beispiel 2 hergestellten Lösung + 100 ml 0,9%ige NaCl-Lösung;
Infusionslösung 2: 50 ml der in Beispiel 2 hergestellten Lösung + 150 ml 0,9%ige NaCl-Lösung;
Infusionslösung 3: 25 ml der in Beispiel 2 hergestellten Lösung + 175 ml 0,9%ige NaCl-Lösung;

Dabei erhielt man jeweils 3 Infusionslösungen mit je 200 ml.

### Beispiel 6: Verabreichen der Infusionslösungen/Infusionskur

In den ersten 10 Behandlungstagen wurde einem Patienten pro Tag 1 Mal die in Beispiel 5 hergestellte Infusionslösung 3 verabreicht. An den Tagen 11 bis 20 wurde eine Behandlungspause eingelegt. An den Tagen 21 bis 30 wurde jeweils 1 Mal am Tag die in Beispiel 5 hergestellte Infusionslösung 2 verabreicht. An den Tagen 31 bis 40 wurde wieder eine Behandlungspause eingelegt. An den Tagen 41 bis 50 wurde ein Mal täglich die erste (Infusionslösung 1) der in Beispiel 5 hergestellten Infusionslösungen verabreicht.

### Beispiel 7: Behandlung von Krebs

20 Krebspatienten (Alter zwischen 21 und 76 Jahren) wurden gemäß der in Beispiel 6 geschilderten 50 tägigen Infusionskur behandelt. Unter den 20 behandelten Krebspatienten waren vier weibliche Patientinnen mit Brusttumoren, ein männlicher Patient mit einem Magentumor, eine weibliche Patientin und 2 männliche Patienten mit Colontumoren, eine weibliche Patientin mit einem Uterustumor, eine weibliche Patientin mit einem Adnextumor, zwei männliche Patienten mit Nierentumoren, ein männlicher Patient mit einem Harnblasentumor, eine weibliche Patientin und 2 männliche Patienten mit Lungenkarzinomen und drei weibliche Patientinnen und ein männlicher Patient mit Leukämie. Bei den Patient(inn)en mit Magenkrebs, Colonkrebs, Uteruskrebs, Adnexkrebs, Nierenkrebs und Harnblasenkrebs wurde die Tumorgröße jeweils vor und nach der Infusionskur durch Sonographie gemessen. Bei den Lungenkrebspatient(inn)en wurde die Tumorgröße vor und nach der Infusionskur durch Röntgenanalyse gemessen. Die Frauen mit Brustkrebs und die Patient(inn)en mit Leukämie wurden vor und nach der Infusionskur durch Palpation untersucht. Bei allen Patienten reduzierte sich die Tumorgröße deutlich. Bei zwei weiblichen Patienten mit Brustkrebs wurde nach der Infusionskur ein um 98 % reduzierte Tumordurchmesser (verglichen mit der Tumorgröße vor der Infusionskur) festgestellt. Bei allen anderen Tumorpatient(inn)en konnte nach der Infusionskur ein zwischen 20 und 95 % reduzierter Tumordurchmesser festgestellt werden. Somit konnte der verbleibende Tumorkern (Nekrose) operativ besser entfernt werden. Bei den Leukämiepatienten wurde eine bis zu 90%ige Reduzierung festgestellt.

## Patentansprüche

1. Zusammensetzung, die Äpfelsäure (A), Kaffeesäure (B), mindestens eine Verbindung aus der Gruppe der Flavan-3-ole (C) und mindestens eine Verbindung aus der Gruppe der Anthocyane (D) umfasst.

2. Zusammensetzung nach Anspruch 1, worin das Flavan-3-ol (C) Catechin und/oder Epicatechin ist.

3. Zusammensetzung nach Anspruch 1 oder 2, worin das Anthocyan (D) Malvidin-3-Glucosid ist.

4. Zusammensetzung nach einem der Ansprüche 1 bis 3, die zusätzlich mindestens eine weitere phenolische Verbindung (E) und/oder eine weitere organische Säure (F), die keine phenolische Verbindung ist, enthält.

5. Zusammensetzung nach einem der Ansprüche 1 bis 4, die zusätzlich mindestens ein Metallsalz (G) der folgenden Metalle enthält: Kalium, Calcium, Kupfer, Eisen, Zink, Magnesium, Natrium und Mangan.

6. Zusammensetzung nach einem der Ansprüche 1 bis 5, die zusätzlich mindestens einen Zucker (H) aus der Gruppe der Mono-, Di- und Oligo-sacharide enthält.

7. Zusammensetzung nach einem der Ansprüche 1 bis 6, worin die Verbindungen (A) bis (D) in den folgenden Mengen vorliegen:
(A) mindestens 30, vorzugsweise 43 bis 73 Gew.-Teile,
(B) mindestens 0.001, vorzugsweise 0.005 bis 0.1 Gew.-Teile,
(C) mindestens 0.005, vorzugsweise 0.015 bis 0.3 Gew.-Teile und
(D) mindestens 0.05, vorzugsweise 0.1 bis 2 Gew.-Teile, bezogen auf 100 Gew.-Teile aller Bestandteile mit Ausnahme von Zucker und ggf. vorhandenem Lösungsmittel.

8. Zusammensetzung gemäß einem der Ansprüche 1 bis 7, **dadurch gekennzeichnet, dass** sie eine pharmazeutische Zusammensetzung ist.

9. Zusammensetzung gemäß Anspruch 8 zur Verwendung in einem Verfahren zur prophylaktischen und therapeutischen Behandlung von Krebs.

10. Verwendung der Zusammensetzung gemäß einem der Ansprüche 1 bis 8 als Nahrungsergänzungsmittel.

11. Nahrungsmittelprodukt, das eine Zusammensetzung nach einem der Ansprüche 1 bis 8 umfasst.

12. Verwendung der Zusammensetzung gemäß einem der Ansprüche 1 bis 8 zur Stabilisierung einer wässrigen Lösung von einem gelösten Alkalisalz des Penicillin G, insbesondere Penicillin G Natrium.

13. Wässrige Lösung, die die Zusammensetzung gemäß einem der Ansprüche 1 bis 8 enthält.

14. Wässrige Lösung gemäß Anspruch 13, die zusätzlich ein gelöstes Alkalisalz des Penicillin G, insbesondere Penicillin G Natrium, enthält.

15. Wässrige Lösung gemäß Anspruch 14 zur Verwendung in einem Verfahren zur prophylaktischen oder therapeutischen Behandlung von bakteriellen Infektionen und/oder einem Verfahren zur prophylaktischen oder therapeutischen Behandlung von Krebs.

## Claims

1. Composition which comprises malic acid (A), caffeic acid (B), at least one compound from the group of flavan-3-ols (C) and at least one compound from the group of anthocyans (D).

2. Composition according to claim 1, wherein the flavan-3-ol (C) is catechol and/or epicatechol.

3. Composition according to claim 1 or 2, wherein the anthocyan (D) is malvidin-3-glucoside.

4. Composition according to one of claims 1 to 3, which additionally contains at least one further phenolic compound (E) and/or one further organic acid (F) which is not a phenolic compound.

5. Composition according to one of claims 1 to 4, which additionally contains at least one metal salt (G) of the following metals: potassium, calcium, copper, iron, zinc, magnesium, sodium and manganese.

6. Composition according to one of claims 1 to 5, which additionally contains at least one sugar (H) from the group of monosaccharides, disaccharides and oligosaccharides.

7. Composition according to one of claims 1 to 6, wherein the compounds (A) to (D) are present in the following quantities:
(A) at least 30, preferably 43 to 73 parts by weight,
(B) at least 0.001, preferably 0.005 to 0.1 parts by weight,
(C) at least 0.005, preferably 0.015 to 0.3 parts by weight and
(D) at least 0.05, preferably 0.1 to 2 parts by weight, based on 100 parts by weight of all constituents with the exception of sugar and optionally present solvent.

8. Composition according to one of claims 1 to 7, **characterised in that** it is a pharmaceutical composition.

9. Composition according to claim 8 for use in a process for the prophylactic and therapeutic treatment of cancer.

10. Use of the composition according to one of claims 1 to 8 as a food supplement.

11. Food product which comprises a composition according to one of claims 1 to 8.

12. Use of the composition according to one of claims 1 to 8 for stabilising an aqueous solution of a dissolved alkali metal salt of penicillin G, in particular sodium penicillin G.

13. Aqueous solution which contains the composition according to one of claims 1 to 8.

14. Aqueous solution according to claim 13, which additionally contains a dissolved alkali metal salt of penicillin G, in particular sodium penicillin G.

15. Aqueous solution according to claim 14 for use in a process for the prophylactic or therapeutic treatment of bacterial infections and/or a process for the prophylactic or therapeutic treatment of cancer

## Revendications

1. Composition, comprenant de l'acide malique (A), de l'acide cafféïque (B), au moins une substance issue du groupe des flavan-3-ole (C) et au moins une substance issue du groupe des anthocyanes (D).

2. Composition selon la revendication 1, dans laquelle le flavan-3-ol (C) est la catéchine et/ou l'épicatechine.

3. Composition selon la revendication 1 ou 2, dans laquelle l'anthocyane (D) est le malvidine-3-glucoside.

4. Composition selon l'une des revendications 1 à 3, contenant en plus au moins une autre substance phénolique (E) et/ou un autre acide organique (F), qui ne soit pas une substance phénolique.

5. Composition selon l'une des revendications 1 à 4, contenant en plus au moins un sel métallique (G) des métaux suivants : potassium, calcium, cuivre, fer, zinc, magnésium, sodium et manganèse.

6. Composition selon l'une des revendications 1 à 5, contenant en plus au moins un sucre (H) issu du groupe des mono-, di- et oligo-saccharides.

7. Composition selon l'une des revendications 1 à 6, dans laquelle les substances (A) à (D) se présentent en les quantités suivantes :
(A) au moins 30, de préférence de 43 à 73 parties en poids,
(B) au moins 0,001, de préférence de 0,005 à 0,1 partie en poids
(C) au moins 0,005, de préférence de 0,015 à 0,3 partie en poids
(D) au moins 0,05, de préférence de 0,1 à 2 parties en poids,
rapporté à 100 parties en poids de tous les constituants, à l'exception du sucre et, le cas échéant, de solvants existants.

8. Composition selon l'une des revendications 1 à 7, **caractérisée en ce qu'**elle contient une composition pharmaceutique.

9. Composition selon la revendication 8, pour utilisation dans un procédé de traitement prophylactique et thérapeutique du cancer.

10. Utilisation de la composition selon l'une des revendications 1 à 8 en tant que produit de complément alimentaire.

11. Produit de complément alimentaire, comprenant une composition selon l'une des revendications 1 à 8.

12. Utilisation de la composition selon l'une des revendications 1 à 8, pour la stabilisation d'une solution aqueuse d'un sel alcalin dissout de la pénicilline G, en particulier la pénicilline G sodique.

13. Solution aqueuse, contenant la composition selon l'une des revendications 1 à 8.

14. Solution aqueuse selon la revendication 13, contenant en plus un sel alcalin dissout de la pénicilline G, en particulier la pénicilline G sodique.

15. Solution aqueuse selon la revendication 14, pour utilisation dans un procédé de traitement prophylactique ou thérapeutique d'infections bactériennes et/ou un procédé de traitement prophylactique ou thérapeutique du cancer.
